# EUROPEAN PATENT SPECIFICATION

(11) **EP 4 355 237 B1**
(45) Date of publication and mention of the grant of the patent: **22.10.2025**
(21) Application number: 22732661.8
(22) Date of filing: 06.06.2022
(51) Int. Cl.: A61B 18/12, A61B 18/14, A61B 34/30, A61B 18/00

(54) **SURGICAL SYSTEMS UTILIZING TEMPERATURE FOR PREDICTING AND/OR CONTROLLING THERMAL SPREAD**
CHIRURGISCHE SYSTEME MIT VORHERSAGE UND/ODER STEUERUNG DER THERMISCHEN AUSBREITUNG ANHAND DER TEMPERATUR
SYSTÈMES CHIRURGICAUX UTILISANT LA TEMPÉRATURE POUR PRÉDIRE ET/OU RÉGULER UNE PROPAGATION THERMIQUE

(30) Priority: 16.06.2021 US 202163211243 P
(43) Date of publication of application: 24.04.2024
(73) Proprietor: Covidien LP, Mansfield, MA 02048 (US)
(72) Inventor: SIMS, Grant T., Boulder, Colorado 80301 (US)
(74) Representative: Maschio & Soames IP Ltd
(86) International application number: PCT/IB2022/055253
(87) International publication number: WO 2022/263971

(56) References cited:
- US-A- 5 769 847
- US-A1- 2020 265 309
- US-A1- 2021 100 606

## Description

### FIELD

The present disclosure relates to surgical systems and methods, and, more particularly, to energy-based surgical systems and methods utilizing temperature to predict and/or control thermal spread.

### BACKGROUND

A surgical forceps is a pliers-like instrument that relies on mechanical action between its jaw members to grasp, clamp, and constrict tissue. Energy-based forceps utilize both mechanical clamping action and energy, e.g., monopolar Radio Frequency (RF), bipolar RF, microwave, ultrasonic, light, thermal, or other suitable energy, to heat tissue to thereby treat, e.g., coagulate, cauterize, or seal, tissue grasped between the jaw members. Typically, once tissue is treated, the surgeon has to accurately sever the treated tissue. Accordingly, many energy-based forceps are designed to incorporate a knife that is advanced between the jaw members to cut the treated tissue. As an alternative to a mechanical knife, an energy-based tissue cutting element may be provided to statically or dynamically cut the treated tissue using energy, e.g., the same or different energy as used for treating the tissue.

While tissue grasped between the jaw members of an electrosurgical forceps is intentionally heated to seal, cut, and/or otherwise treat the tissue, it is at the same time desirable to minimize or inhibit thermal spread, the heating of tissue external to the jaw members as a side effect of the application of energy to tissue grasped between the jaw members.

US 2020/265309 discloses estimating tissue parameters using surgical devices.

### SUMMARY

As used herein, the term "distal" refers to the portion that is being described which is farther from an operator (whether a human surgeon or a surgical robot), while the term "proximal" refers to the portion that is being described which is closer to the operator. Terms including "generally," "about," "substantially," and the like, as utilized herein, are meant to encompass variations, e.g., manufacturing tolerances, material tolerances, use and environmental tolerances, measurement variations, and/or other variations, up to and including plus or minus 10 percent. Further, any or all of the aspects described herein, to the extent consistent, may be used in conjunction with any or all of the other aspects described herein.

The invention is defined by the appended claims. Provided in accordance with aspects of the present disclosure is an electrosurgical system including an end effector assembly, at least one temperature sensor, and an electrosurgical generator. The end effector assembly includes first and second jaw members each defining an electrically-conductive tissue-contacting surface. At least one of the first or second jaw members is movable relative to the other between a spaced-apart position and an approximated position for grasping tissue between the tissue-contacting surfaces thereof. The at least one temperature sensor is associated with the end effector assembly. The electrosurgical generator is electrically coupled to the tissue-contacting surfaces and configured to supply electrosurgical energy thereto for treating tissue grasped between the tissue-contacting surfaces. The electrosurgical generator is coupled to the at least one temperature sensor and configured to receive sensed temperature data therefrom. The electrosurgical generator includes a controller configured, in real time, to control the supply of electrosurgical energy to tissue, predict thermal spread beyond the first and second jaw members based at least on the sensed temperature data, modify, where it is determined that the predicted thermal spread is above a threshold thermal spread, the supply of electrosurgical energy to tissue to inhibit realization of the predicted thermal spread.

In an aspect of the present disclosure, the at least one temperature sensor is disposed on one of the first or second jaw members. More specifically, the at least one temperature sensor may be disposed adjacent an outer periphery of the first and/or second jaw members, inwardly spaced from the outer periphery, or disposed in any other suitable position.

In another aspect of the present disclosure, the at least one temperature sensor is disposed on a device independent of the end effector assembly.

In yet another aspect of the present disclosure, the controller is configured to predict the thermal spread based at least on the sensed temperature data, electrical data associated with the supply of energy, e.g., impedance, and/or progression status information.

In still another aspect of the present disclosure, the controller is configured to modify the supply of electrosurgical energy to tissue, where it is determined that the predicted thermal spread is above the threshold thermal spread, by reducing power, switching an energy-delivery algorithm, or stopping the supply of energy. The threshold thermal spread may be input by the user, determined from static information (such as patient information, procedure information, instrument information, tissue information, etc.), and/or determined from dynamic information (such as any of the feedback information detailed herein).

In still yet another aspect of the present disclosure, the controller incorporates or is configured to communicate with a machine learning algorithm configured to facilitate the prediction of thermal spread. In aspects, machine learning may be utilized to tune the algorithm based on the specific thermal measurements, patient tissue data, and/or other input to feedback data to achieve a constant amount of thermal spread within acceptable limits.

In another aspect of the present disclosure, the controller is configured to predict thermal spread at least 3 seconds into the future and/or to output, for display, an indication to the user of a margin of predicted thermal spread (such as, for example, overlaid over a video image of the surgical site to visually indicate the margins relative to the end effector assembly and tissue).

A method, not part of the claimed invention as such but useful for the general understanding of the invention, of treating tissue provided in accordance with the present disclosure includes grasping tissue between tissue-contacting surfaces of first and second jaw members of an end effector assembly, controlling the supply of electrosurgical energy from the tissue-contacting surfaces to the tissue grasped therebetween, sensing temperature at or adjacent to the end effector assembly, predicting, in real time, thermal spread beyond the first and second jaw members based at least on the sensed temperature, and modifying, where it is determined that the predicted thermal spread is above a threshold thermal spread, the supply of electrosurgical energy to tissue to inhibit realization of the predicted thermal spread.

In an aspect of the present disclosure, sensing the temperature includes sensing a temperature of at least one of the first or second jaw members. More specifically, in aspects, the temperature of at least one of tissue-contacting surfaces may be sensed.

In another aspect of the present disclosure, sensing the temperature includes sensing a temperature of the tissue grasped between the tissue-contacting surfaces and/or sensing a temperature of tissue adjacent to the end effector assembly.

In still another aspect of the present disclosure, predicting the thermal spread is based at least on the sensed temperature data and electrical data, e.g., impedance data, associated with the supply of energy.

In yet another aspect of the present disclosure, predicting the thermal spread is based at least on the sensed temperature data and progression status information. The sensed temperature data and/or predicted thermal spread, in aspects, may also be utilized to predict confidence in the reliability of a tissue seal. For example, a low confidence may be determined where the temperature is too high (above an overall threshold or a threshold for the particular progression status) and/or the predicted thermal spread is too great (above an overall threshold or a threshold for the particular progression status); likewise, a low confidence may be determined where the temperature is too low (below an overall threshold or a threshold for the particular progression status) and/or the predicted thermal spread is too low (below an overall threshold or a threshold for the particular progression status). A high confidence may be determined where the temperature and/or predicted thermal spread are within acceptable limits.

In still yet another aspect of the present disclosure, modifying the supply of electrosurgical energy includes reducing power, switching an energy-delivery algorithm, or stopping the supply of energy.

In another aspect of the present disclosure, the controller is configured to predict thermal spread at least 3 seconds into the future.

### BRIEF DESCRIPTION OF DRAWINGS

The above and other aspects and features of the present disclosure will become more apparent in view of the following detailed description when taken in conjunction with the accompanying drawings wherein like reference numerals identify similar or identical elements.
FIG. 1 is a perspective view of a shaft-based electrosurgical forceps provided in accordance with the present disclosure connected to an electrosurgical generator;
FIG. 2A is a perspective view of a distal end portion of the forceps of FIG. 1, wherein jaw members of the end effector assembly of the forceps are disposed in a spaced-apart position;
FIG. 2B is a perspective view of the distal end portion of the forceps of FIG. 2A, wherein the jaw members are disposed in an approximated position;
FIG. 3 is a perspective view of a hemostat-style electrosurgical forceps provided in accordance with the present disclosure;
FIG. 4 is a schematic illustration of a robotic surgical instrument provided in accordance with the present disclosure;
FIG. 5 is a block diagram of the electrosurgical generator of FIG. 1;
FIG. 6 is a block diagram of a controller of the electrosurgical generator of FIG. 5;
FIG. 7 is a logic diagram of a machine learning algorithm in accordance with the present disclosure;
FIGS. 8 and 9 are transverse, cross-sectional views of the jaw members of the end effector assembly of FIG. 2A shown grasping tissue therebetween and including different configurations of at least one temperature sensor incorporated therein; and
FIG. 10 is a transverse, cross-sectional view of the jaw members of the end effector assembly of FIG. 2A shown grasping tissue therebetween and including an external temperature sensing device associated therewith.

### DETAILED DESCRIPTION

The present disclosure provides energy-based surgical systems and methods utilizing temperature for predicting and/or controlling thermal spread. Various exemplary energy-based surgical instruments and systems are detailed below; however, the aspects and features of the present disclosure are not limited thereto as any other suitable energy-based surgical instruments and/or systems are also contemplated for use in accordance with the present disclosure.

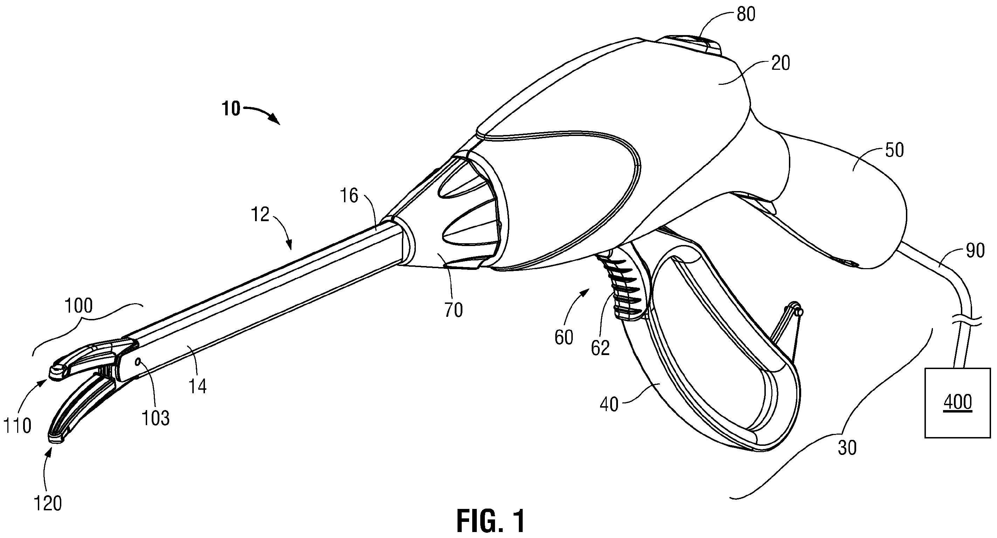

Referring to FIG. 1, a shaft-based electrosurgical forceps provided in accordance with the present disclosure is shown generally identified by reference numeral 10. Aspects and features of forceps 10 not germane to the understanding of the present disclosure are omitted to avoid obscuring the aspects and features of the present disclosure in unnecessary detail.

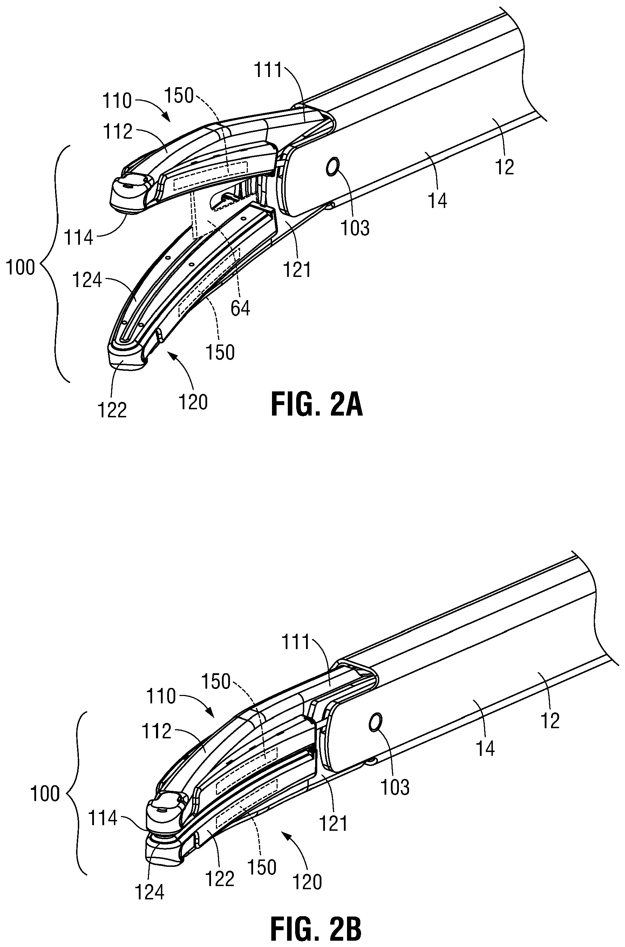

Forceps 10 includes a housing 20, a handle assembly 30, a trigger assembly 60, a rotating assembly 70, an activation switch 80, and an end effector assembly 100. Forceps 10 further includes a shaft 12 having a distal end portion 14 configured to (directly or indirectly) engage end effector assembly 100 and a proximal end portion 16 that (directly or indirectly) engages housing 20. Forceps 10 also includes cable 90 that connects forceps 10 to an electrosurgical generator 400. Cable 90 includes a wire (or wires) (not shown) extending therethrough that has sufficient length to extend through shaft 12 in order to provide energy to one or both tissue-contacting surfaces 114, 124 of jaw members 110, 120, respectively, of end effector assembly 100 (see FIGS. 2A and 2B). In aspects, bipolar Radio Frequency (RF) is conducted between tissue-contacting surfaces 114, 124 of jaw members 110, 120, respectively, by energizing tissue-contacting surfaces 114, 124 to different potentials to establish a potential gradient therebetween, although other suitable energy configurations and/or energy modalities are also contemplated. Activation switch 80 is coupled to tissue-contacting surfaces 114, 124 (FIGS. 2A and 2B) and electrosurgical generator 400 for enabling the selective activation of the supply of energy to jaw members 110, 120 for treating, e.g., sealing, tissue.

Handle assembly 30 of forceps 10 includes a fixed handle 50 and a movable handle 40 (although both handles 40, 50 may move, in aspects). Fixed handle 50 is integrally associated with housing 20 and handle 40 is movable relative to fixed handle 50. Movable handle 40 of handle assembly 30 is operably coupled to a drive assembly (not shown) that, together, mechanically cooperate to impart movement of one or both of jaw members 110, 120 of end effector assembly 100 about a pivot 103 between a spaced-apart position (FIG. 2A) and an approximated position (FIG. 2B) to grasp tissue between jaw members 110, 120. As shown in FIG. 1, movable handle 40 is initially spaced-apart from fixed handle 50 and, correspondingly, jaw members 110, 120 of end effector assembly 100 are disposed in the spaced-apart position. Movable handle 40 is depressible from this initial position to a depressed position corresponding to the approximated position of jaw members 110, 120 (FIG. 2B).

Trigger assembly 60 includes a trigger 62 coupled to housing 20 and movable relative thereto between an un-actuated position and an actuated position. Trigger 62 is operably coupled to a knife 64 (FIG. 2A), so as to actuate knife 64 (FIG. 2A) to cut tissue grasped between jaw members 110, 120 of end effector assembly 100 upon actuation of trigger 62. As an alternative to knife 64, other suitable mechanical, electrical, or electromechanical cutting mechanisms (stationary or movable) are also contemplated.

With additional reference to FIGS. 2A and 2B, end effector assembly 100, as noted above, includes first and second jaw members 110, 120. Each jaw member 110, 120 includes a proximal flange portion 111, 121, an outer insulative jaw housing 112, 122 disposed about the distal portion (not explicitly shown) of each jaw member 110, 120, and a tissue-contacting surface 114, 124, respectively. Proximal flange portions 111, 121 are pivotably coupled to one another about pivot 103 for moving jaw members 110, 120 between the spaced-apart and approximated positions, although other suitable mechanisms for pivoting jaw members 110, 120 relative to one another are also contemplated. The distal portions of jaw members 110, 120 are configured to support jaw housings 112, 122, and tissue-contacting surfaces 114, 124, respectively, thereon.

Outer insulative jaw housings 112, 122 of jaw members 110, 120 support and retain tissue-contacting surfaces 114, 124 on respective jaw members 110, 120 in opposed relation relative to one another. Tissue-contacting surfaces 114, 124 are at least partially formed from an electrically conductive material, e.g., for conducting electrical energy therebetween for treating tissue, although tissue-contacting surfaces 114, 124 may alternatively be configured to conduct any suitable energy, e.g., thermal, microwave, light, ultrasonic, etc., through tissue grasped therebetween for energy-based tissue treatment. As mentioned above, tissue-contacting surfaces 114, 124 are coupled to activation switch 80 and electrosurgical generator 400, e.g., via the wires extending from cable 90 through forceps 10, such that energy may be selectively supplied to tissue-contacting surface 114 and/or tissue-contacting surface 124 and conducted therebetween and through tissue disposed between jaw members 110, 120 to treat tissue.

Continuing with reference to FIGS. 2A and 2B, end effector assembly 100 further includes at least one temperature sensor 150 disposed within, on, or otherwise associated with one or both of jaw members 110, 120. Temperature sensor(s) 150, based on its location and/or configuration, senses: a temperature of one or both of tissue-contacting surfaces 114, 124; a temperature of tissue disposed between tissue-contacting surfaces 114, 124; a temperature of a peripheral edge(s) of one or both of jaw members 110, 120; a temperature of an exterior surface of one or both of jaw members 110, 120; a temperature of an exterior environment adjacent one or both of jaw members 110, 120; and/or a temperature of tissue exterior of and adjacent to one or both of jaw members 110, 120. Temperature sensor(s) 150 are further configured to provide feedback to generator 400 (FIG. 1) to enable generator 400 (FIG. 1) to sense temperature for use in predicting and/or controlling thermal spread, as detailed below.

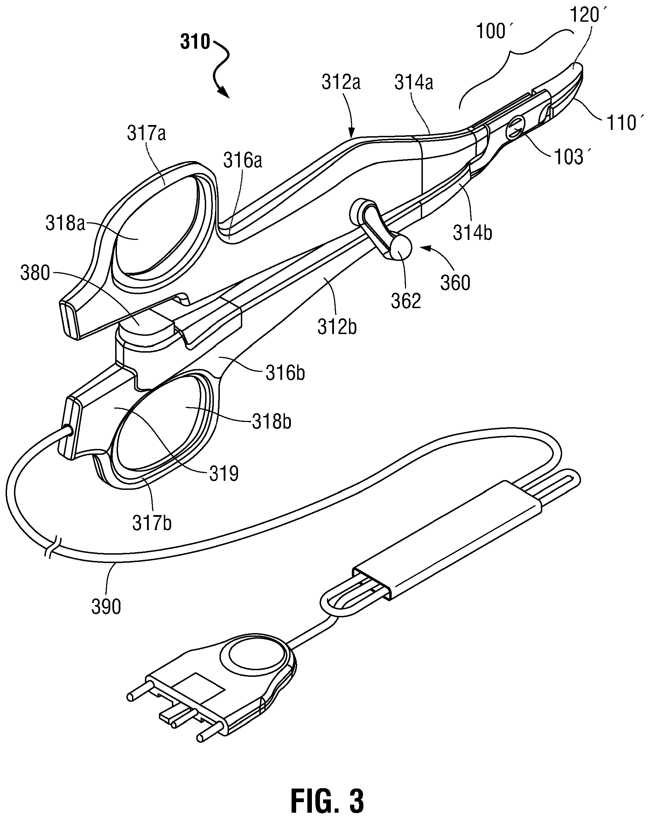

Referring to FIG. 3, a hemostat-style electrosurgical forceps provided in accordance with the present disclosure is shown generally identified by reference numeral 310. Aspects and features of forceps 310 not germane to the understanding of the present disclosure are omitted to avoid obscuring the aspects and features of the present disclosure in unnecessary detail.

Forceps 310 includes two elongated shaft members 312a, 312b, each having a proximal end portion 316a, 316b, and a distal end portion 314a, 314b, respectively. Forceps 310 is configured for use with an end effector assembly 100' similar to end effector assembly 100 (FIGS. 2A and 2B). More specifically, end effector assembly 100' includes first and second jaw members 110', 120' attached to respective distal end portions 314a, 314b of shaft members 312a, 312b. Jaw members 110', 120' are pivotably connected about a pivot 103'. Each shaft member 312a, 312b includes a handle 317a, 317b disposed at the proximal end portion 316a, 316b thereof. Each handle 317a, 317b defines a finger hole 318a, 318b therethrough for receiving a finger of the user. As can be appreciated, finger holes 318a, 318b facilitate movement of the shaft members 312a, 312b relative to one another to, in turn, pivot jaw members 110', 120' from the spaced-apart position, wherein jaw members 110', 120' are disposed in spaced relation relative to one another, to the approximated position, wherein jaw members 110', 120' cooperate to grasp tissue therebetween.

One of the shaft members 312a, 312b of forceps 310, e.g., shaft member 312b, includes a proximal shaft connector 319 configured to connect forceps 310 to electrosurgical generator 400 (FIG. 1). Proximal shaft connector 319 secures a cable 390 to forceps 310 such that the user may selectively supply energy to jaw members 110', 120' for treating tissue. More specifically, an activation switch 380 is provided for supplying energy to jaw members 110', 120' to treat tissue upon sufficient approximation of shaft members 312a, 312b, e.g., upon activation of activation switch 380 via shaft member 312a.

Forceps 310 further includes a trigger assembly 360 including a trigger 362 coupled to one of the shaft members, e.g., shaft member 312a, and movable relative thereto between an un-actuated position and an actuated position. Trigger 362 is operably coupled to a knife (not shown; similar to knife 64 (FIG. 2A) of forceps 10 (FIG. 1)) so as to actuate the knife to cut tissue grasped between jaw members 110', 120' of end effector assembly 100' upon movement of trigger 362 to the actuated position. Similarly as noted above with respect to forceps 10 (FIG. 1), other suitable cutting mechanisms are also contemplated.

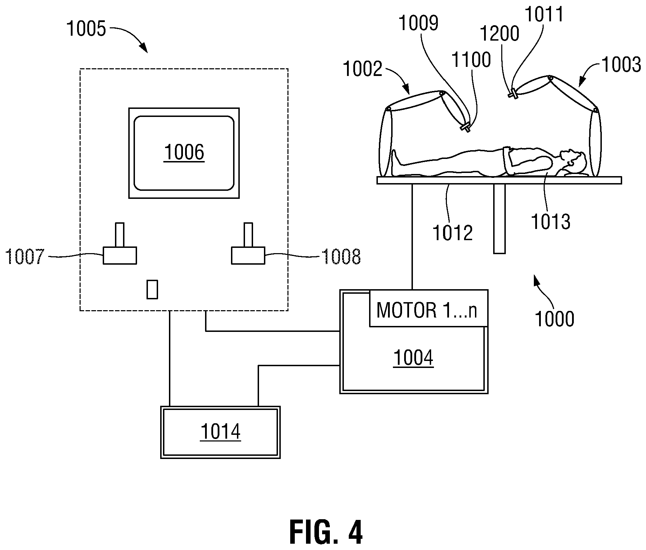

Referring to FIG. 4, a robotic surgical instrument provided in accordance with the present disclosure is shown generally identified by reference numeral 1000. Aspects and features of robotic surgical instrument 1000 not germane to the understanding of the present disclosure are omitted to avoid obscuring the aspects and features of the present disclosure in unnecessary detail.

Robotic surgical instrument 1000 includes a plurality of robot arms 1002, 1003; a control device 1004; and an operating console 1005 coupled with control device 1004. Operating console 1005 may include a display device 1006, which may be set up in particular to display three-dimensional images; and manual input devices 1007, 1008, by means of which a surgeon may be able to telemanipulate robot arms 1002, 1003 in a first operating mode. Robotic surgical instrument 1000 may be configured for use on a patient 1013 lying on a patient table 1012 to be treated in a minimally invasive manner. Robotic surgical instrument 1000 may further include a database 1014, in particular coupled to control device 1004, in which are stored, for example, preoperative data from patient 1013 and/or anatomical atlases.

Each of the robot arms 1002, 1003 may include a plurality of members, which are connected through joints, and an attaching device 1009, 1011, to which may be attached, for example, an end effector assembly 1100, 1200, respectively. End effector assembly 1100 is similar to end effector assembly 100 (FIGS. 2A and 2B), although other suitable end effector assemblies for coupling to attaching device 1009 are also contemplated. End effector assembly 1100 is connected to electrosurgical generator 400 (FIG. 1), which may be integrated into or separate from robotic surgical instrument 1000. End effector assembly 1200 may be any end effector assembly, e.g., an endoscopic camera, other surgical tool, etc. Robot arms 1002, 1003 and end effector assemblies 1100, 1200 may be driven by electric drives, e.g., motors, that are connected to control device 1004. Control device 1004 (e.g., a computer) may be configured to activate the motors, in particular by means of a computer program, in such a way that robot arms 1002, 1003, their attaching devices 1009, 1011, and end effector assemblies 1100, 1200 execute a desired movement and/or function according to a corresponding input from manual input devices 1007, 1008, respectively. Control device 1004 may also be configured in such a way that it regulates the movement of robot arms 1002, 1003 and/or of the motors.

Referring to FIG. 1-2B and 5, electrosurgical generator 400 is shown as a schematic block diagram. Generator 400 may be utilized as a stand-alone generator (as shown in FIG. 1), may be incorporated into a surgical instrument 10, 310, 1000 (FIGS. 1, 3, and 4, respectively), or may be provided in any other suitable manner. Generator 400 includes sensor circuitry 422, a controller 424, a high voltage DC power supply ("HVPS") 426 and an RF output stage 428. Sensor circuitry 422 is configured to receive sensor feedback, e.g., feedback representative of temperature from temperature sensor(s) 150, and to relay the same to controller 424. Voltage, current, power, impedance, and/or other electrical feedback associated with the delivery of energy, e.g., bipolar RF energy, to tissue may also be communicated to controller 424 to control the energy output. Further, controller 424 may implement and track the progression of energy-delivery (e.g., for sealing tissue), and/or the progression of an implemented energy-delivery algorithm (e.g., a tissue sealing algorithm), such as, for example, by determining a stage of the tissue sealing algorithm, a time remaining to completion of tissue sealing, etc. This progression status information related to tissue sealing (or other tissue treatment) may be utilized for various purposes, such as detailed below.

Controller 424 is configured to control the output of energy from HVPS 426 to RF output stage 428 and, thus, the application of energy from tissue-contacting surfaces 114, 124 of jaw members 110, 120 to tissue grasped therebetween. HVPS 426, under the direction of controller 424, provides high voltage DC power to RF output stage 428 which converts the high voltage DC power into bipolar RF energy for delivery to tissue-contacting 114, 124 of jaw members 110, 120, respectively, of end effector assembly 100 (see FIGS. 2A and 2B). In particular, RF output stage 428 generates sinusoidal waveforms of high frequency RF energy. RF output stage 428 may be configured to generate waveforms having various duty cycles, peak voltages, crest factors, and other properties. Other suitable configurations are also contemplated such as for example, pulsed energy output, other waveforms, etc.

In aspects, controller 424 of generator 400 may control energy delivery in accordance with a tissue sealing algorithm to seal tissue grasped between tissue-contacting 114, 124 of jaw members 110, 120, respectively, of end effector assembly 100 such as, for example, the tissue sealing algorithm detailed in U.S. Patent No. 9,186,200 entitled "System and Method for Tissue Sealing" and issued on November 17, 2015, or the tissue sealing algorithm detailed in U.S. Patent No. 10,617,463 entitled "System and Method for Controlling Power in an Electrosurgical Generator" and issued on April 14, 2020.

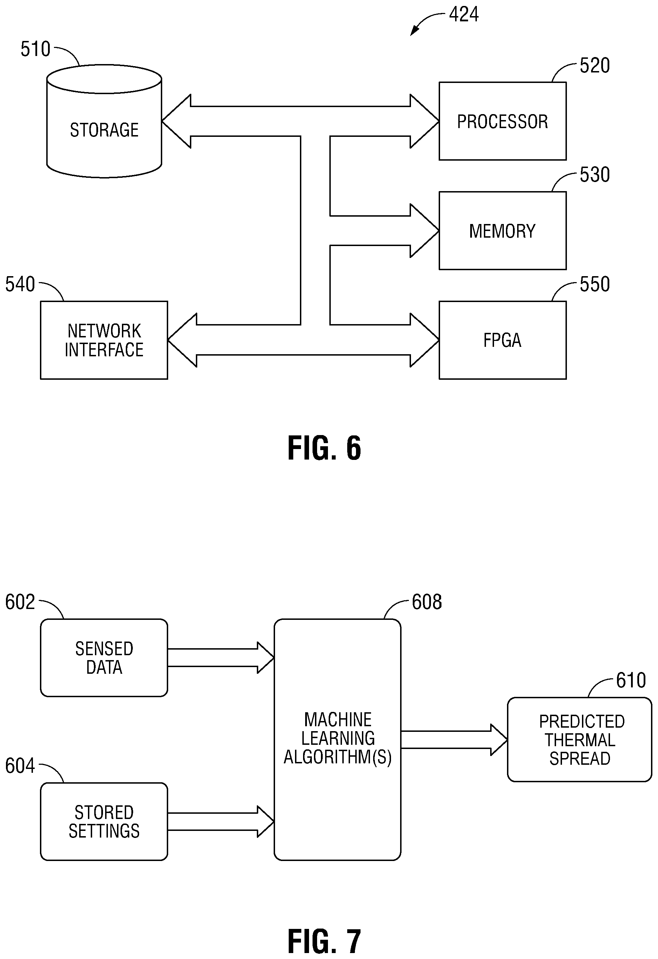

With additional reference to FIG. 6, controller 424 includes a processor 520 connected to a computer-readable storage medium or a memory 530 which may be a volatile type memory, e.g., RAM, or a non-volatile type memory, e.g., flash media, disk media, etc. In aspects, processor 520 may be, without limitation, a digital signal processor, a microprocessor, an ASIC, a graphics processing unit (GPU), field-programmable gate array (FPGA), or a central processing unit (CPU). Memory 530 can be random access memory, read-only memory, magnetic disk memory, solid state memory, optical disc memory, and/or another type of memory. In aspects, memory 530 can be separate from controller 424 and can communicate with processor 520 through communication buses of a circuit board and/or through communication cables such as serial ATA cables or other types of cables. Memory 530 includes computer-readable instructions that are executable by processor 520 to operate controller 424. Controller 424 includes a network interface 540 to communicate with other computers or a server. In aspects, a storage device 510 may be used for storing data. In aspects, controller 424 may include one or more FPGAs 550. FPGA 550 may be used for executing various algorithms, e.g., fixed algorithms, machine learning algorithms, etc.

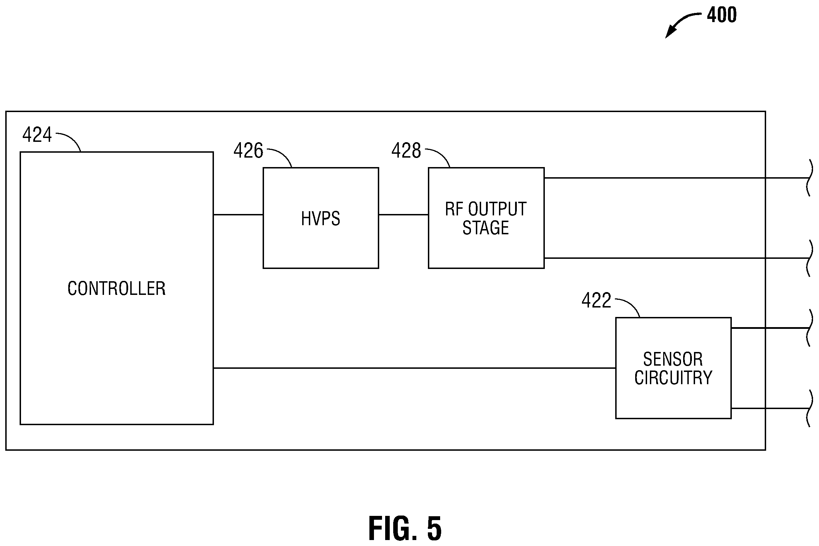

Memory 530 stores suitable instructions, to be executed by processor 520, for receiving the sensed data, e.g., sensed temperature data from sensor circuitry 422 (FIG. 5) and/or any other suitable feedback or other data, accessing storage device 510 of controller 424, and predicting thermal spread based upon the sensed data and/or other data, in real time. Memory 530 further stores suitable instructions, to be executed by processor 520, to provide an alert to the user, e.g., an audible alert (such as a tone), a visual alert (such as a flashing light), and/or a tactile alert (such as vibration of housing 20 (FIG. 1)), based upon the predicted thermal spread and/or to control energy delivery based thereon. Although illustrated as part of generator 400, it is also contemplated that controller 424 be remote from generator 400, e.g., on a remote server, and accessible by generator 400 via a wired or wireless connection. In configurations where controller 424 is remote, it is contemplated that controller 424 may be accessible by and connected to multiple generators 400.

Controller 424, more specifically, is configured to receive the sensor feedback from sensor circuitry 422 regarding the sensed temperature(s) and/or to receive or otherwise utilize other feedback data (including, for example, the progression status information, electrical feedback data, etc.) to predict thermal spread in real time during the application of energy to tissue. Based on the predicted thermal spread, controller 424 may output a suitable alert to the user indicating that a potentially unacceptable amount of thermal spread (e.g., an amount of thermal spread exceeding a thermal spread threshold in volumetric size, distance from jaw members 110, 120, and/or temperature) has been predicted. Additionally or alternatively, controller 424 may be configured to automatically control the output of energy from HVPS 426 to RF output stage 428 such that thermal spread can be avoided or minimized (in volumetric size, distance from jaw members 110, 120, and/or temperature) to below a threshold amount of thermal spread. That is, rather than determining a prior, present, or immediate future amount of thermal spread (which may thus be unavoidable and/or irreversible), controller 424 is configured to predict the progression of thermal spread sufficiently far into the future (e.g., 1 second, 3 seconds, 5 seconds, etc. in the future) to enable, in instances where an unacceptable amount of thermal spread is predicted, an alert to be provided to the user for the user modify the tissue treatment, and/or to control energy-delivery automatically (e.g., to control energy-delivery parameters, stop energy delivery altogether, etc.). If the predicted thermal spread is within acceptable limits, the energy-delivery may continue without modification. In this manner, predicted unacceptable thermal spread can be avoided before unintended tissue damage occurs. It is noted that the prediction of thermal spread need not be made with regard to a pre-set or pre-determined future time but, rather, the future time may be dynamically selected based on, for example, an estimated sealing time remaining, the tissue treatment being performed, etc.

Predicting thermal spread, as mentioned above, is based on temperature data and/or other feedback data such as, for example, electrical feedback data (e.g., voltage, current, power, impedance, etc.), the progression status information, etc. The other feedback data may also include, for example, tissue mass data, jaw angle data, tissue type data, etc. Non-feedback data such as, for example, retrieved or input patient data, retrieved or input data relating to the procedure to be performed, etc. may also be utilized. Further, coefficient data may be stored, obtained, or otherwise provided for utilization such as, for example, thermal conductivity of tissue (in aspects, of the particular tissue type, tissue mass, etc. identified), thermal conductivity of the particular end effector assembly 100 (FIG. 1) utilized, etc. The temperature data, electrical data, and/or other data may be snapshot data (e.g., at a particular moment in time) or may be trend data (e.g., changing over time). In aspects, as noted above, the temperature data may be temperature data associated with the outer periphery of jaw members 110, 120 (see FIGS. 2A and 2B) and/or the environment and/or tissue adjacent thereto; in other aspects, the temperature data may be temperature data associated with tissue-contacting surfaces 114, 124 of jaw members 110, 120 (see FIGS. 2A and 2B) and/or tissue disposed therebetween. In aspects, at least the temperature data and impedance data are utilized to predict thermal spread. In additional or alternative aspects, the progression status information is utilized in the prediction of thermal spread by enabling determination of whether thermal spread and/or the rate thereof is likely to increase, decrease, or stay the same based on the present and/or upcoming expected energy delivery parameters or energy delivery stage(s). For example, even where the present temperature, thermal spread, and/or other conditions are the same, the predicted thermal spread may be different in a situation where tissue treatment is close to completion or where a drop off in energy application (or thermal conductivity of tissue) is expected, as compared to a situation where an increase in energy application (or thermal conductivity of tissue) is expected.

Regardless of the particular data utilized, thermal spread may be predicted, for example, by plotting the data along one or more curves, utilizing one or more look-up tables, matching the data to empirical or theoretical (e.g., modeled) data and the corresponding results, inputting the data into a one or more fixed algorithms, and/or in any other suitable manner or combinations of manners. In aspects, machine learning is utilized to predict or facilitate the prediction of thermal spread, as detailed below.

With reference to FIGS. 6 and 7, in aspects where one or more machine learning machine learning algorithms 608 are used, storage device 510 of controller 424 stores the one or more machine learning algorithms 608. The machine learning algorithm(s) 608 may be trained on and learn from stored settings 604, e.g., theoretical data, empirical data, and/or other data initially input into the one or more machine learning applications, and/or the sensed data 602 from sensor circuitry 422 (FIG. 5) (or other sensed, feedback, or input data) in order to enable the machine learning application(s) to output a prediction of thermal spread 610. In aspects, training the machine learning algorithm may be performed by a computing device outside of generator 400 and the resulting algorithm may be communicated to controller 424 of generator 400.

Turning back to FIGS. 1-2B, 5, and 6, in aspects, controller 424 utilizes the predicted thermal spread and communicates the same to a computing device, e.g., of controller 424, for use in controlling the output of energy from HVPS 426 to RF output stage 428. This controlling may include starting, continuing, modifying, or stopping the output of energy. More specifically: a tissue treating algorithm stored in storage device 510 of controller 424 may be implemented, modified, stopped, switched to another tissue treating algorithm, etc.; the waveform output may be modified, stopped, switched to another tissue treating waveform; a setting may be changed, e.g., power may be increased or decreased; and/or an energy output time may be increased or decreased. That is, the energy output is adapted, e.g., reduced, if necessary, in accordance with the predicted thermal spread. In this manner, undesirable predicted thermal spread can be avoided before any potential tissue damage occurs or to minimize potential tissue damage. Where the predicted thermal spread is within acceptable limits, the output of energy from HVPS 426 to RF output stage 428 may be unchanged. In other aspects, where the predicted thermal spread is within acceptable limits (and sufficiently minimal), the output of energy from HVPS 426 to RF output stage 428 may be modified, e.g., increased, to, for example, expedite the tissue treatment.

The particular control to be implemented, if deemed necessary based on the predicted thermal spread, may be determined, for example, by plotting the data along one or more curves, utilizing one or more look-up tables, matching the data to empirical or theoretical (e.g., modeled) data and the corresponding results, inputting the data into a one or more fixed algorithms, utilizing one or more machine learning algorithms, and/or in any other suitable manner or combinations of manners.

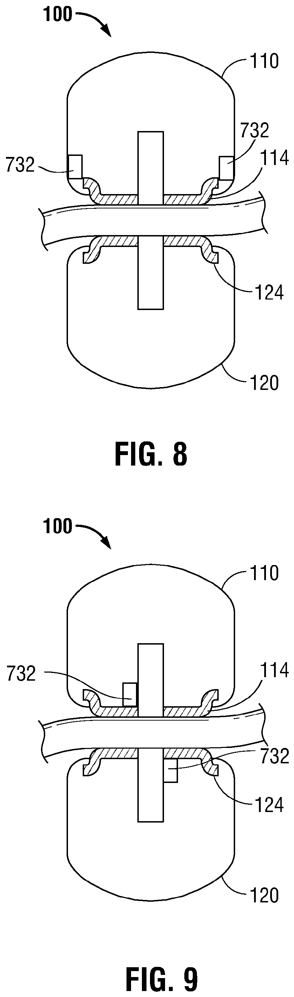

Turning to FIGS. 8-10, detailed below are aspects of temperature sensor configurations incorporated into or associated with jaw member 110 and/or jaw member 120 of end effector assembly 100 (or any other end effector assembly detailed herein or suitable for use in accordance with the present disclosure) for providing the temperature data for use in predicting thermal spread. More specifically, as detailed above, the temperature sensor(s) is configured to communicate sensor feedback to sensor circuitry 422 of generator 400 (see FIG. 5) which, in turn, is configured to communicate with controller 424 (see FIG. 6) to enable the prediction of thermal spread in real time during the application of energy to tissue.

Referring to FIG. 8, in aspects, first and second temperature sensors 732 are disposed on opposing lateral sides of jaw member 110, although additional or alternate temperature sensors 732 may be disposed on opposing lateral sides of jaw member 120 and/or only one temperature sensor 732 on one lateral side (of either or both jaw members 110, 120 on the same or different sides) may be provided. Temperature sensors 732, positioned in this manner, enable sensing of temperatures of the peripheral edges of jaw members 110, 120; a temperature of an exterior surface of one or both of jaw members 110, 120; a temperature of an exterior environment adjacent one or both of jaw members 110, 120, and/or a temperature of tissue exterior of and adjacent to one or both of jaw members 110, 120.

With reference to FIG. 9, in aspects, first and second temperature sensors 732 are disposed on jaw members 110, 120 and associated with respective tissue-contacting surfaces 114, 124 thereof, although a temperature sensor 732 may be disposed on only one of jaw members 110, 120. Temperature sensors 732, positioned in this manner, enable sensing of a temperature of one or both of tissue-contacting surfaces 114, 124 and/or a temperature of tissue disposed between tissue-contacting surfaces 114, 124.

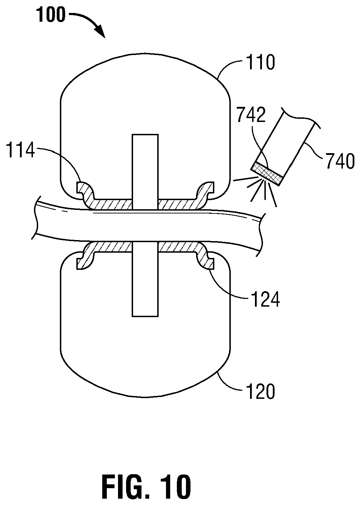

As shown in FIG. 10, temperature sensing may be provided by a device 740 external and independent of end effector assembly 100. Device 740 may be, for example, a thermal camera, infrared scanner, or other suitable temperature sensing device including one or more temperature sensors 742. Temperature sensor(s) 742 may be positioned to enable sensing of a temperature of: the peripheral edge(s) of jaw member(s) 110, 120; a temperature of an exterior surface of end effector assembly 100; a temperature of an exterior environment adjacent end effector assembly 100; and/or a temperature of tissue exterior of and adjacent to end effector assembly 100. Other suitable locations and/or configurations of temperature sensors, including combinations of the above and/or other suitable temperature sensors, whether incorporated into end effector assembly 100 or remote therefrom, are also contemplated.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structures or any other physical structure suitable for implementation of the described techniques. Also, the techniques could be fully implemented in one or more circuits or logic elements.

While several aspects of the disclosure have been shown in the drawings, it is not intended that the disclosure be limited thereto. Therefore, the above description should not be construed as limiting, but merely as exemplifications of particular configurations. Those skilled in the art will envision other modifications within the scope of the claims appended hereto.

## Claims

1. An electrosurgical system, comprising:
an end effector assembly (100) including first (110) and second (120) jaw members each defining an electrically-conductive tissue-contacting surface (114, 124), at least one of the first or second jaw members (110, 120) movable relative to the other between a spaced-apart position and an approximated position for grasping tissue between the tissue-contacting surfaces (114, 124) thereof;
at least one temperature sensor (150) associated with the end effector assembly (100); and
an electrosurgical generator (400) electrically coupled to the tissue-contacting surfaces (114, 124) and configured to supply electrosurgical energy thereto for treating tissue grasped between the tissue-contacting surfaces (114, 124), the electrosurgical generator coupled to the at least one temperature sensor and configured to receive sensed temperature data therefrom, wherein the electrosurgical generator includes a controller (424) configured, in real time, to
control the supply of electrosurgical energy to tissue;
predict thermal spread beyond the first and second jaw members based at least on the sensed temperature data; and
modify, where it is determined that the predicted thermal spread is above a threshold thermal spread, the supply of electrosurgical energy to tissue to inhibit realization of the predicted thermal spread.

2. The electrosurgical system according to claim 1, wherein the at least one temperature sensor is disposed on one of the first or second jaw members.

3. The electrosurgical system according to claim 2, wherein the at least one temperature sensor is disposed adjacent an outer periphery of the at least one of the first or second jaw members.

4. The electrosurgical system according to claim 2, wherein the at least one temperature sensor is inwardly-spaced from an outer periphery of the at least one of the first or second jaw members.

5. The electrosurgical system according to claim 1, wherein the at least one temperature sensor is disposed on a device independent of the end effector assembly.

6. The electrosurgical system according to claim 1, wherein the controller is configured to predict the thermal spread based at least on the sensed temperature data and electrical data associated with the supply of energy, optionally wherein the electrical data at least includes impedance data.

7. The electrosurgical system according to claim 1, wherein the controller is configured to predict the thermal spread based at least on the sensed temperature data and progression status information.

8. The electrosurgical system according to claim 1, wherein the controller is configured to modify the supply of electrosurgical energy to tissue, where it is determined that the predicted thermal spread is above the threshold thermal spread, by reducing power, switching an energy-delivery algorithm, or stopping the supply of energy.

9. The electrosurgical system according to claim 1, wherein the controller incorporates or is configured to communicate with a machine learning algorithm configured to facilitate the prediction of thermal spread.

10. The electrosurgical system according to claim 1, wherein the controller is configured so that the prediction of the thermal spread is for at least 3 seconds into the future.

## Patentansprüche

1. Elektrochirurgisches System, das Folgendes umfasst:
eine Endeffektoranordnung (100), die ein erstes (110) und ein zweites (120) Backenelement beinhaltet, die jeweils eine elektrisch leitfähige Gewebekontaktfläche (114, 124) definieren,
wobei mindestens eines der ersten oder zweiten Backenelemente (110, 120) relativ zu dem anderen zwischen einer beabstandeten Position und einer angenäherten Position zum Greifen von Gewebe zwischen den Gewebekontaktflächen (114, 124) davon beweglich ist;
mindestens einen Temperatursensor (150), der mit der Endeffektoranordnung (100) assoziiert ist; und
einen elektrochirurgischen Generator (400), der elektrisch mit den Gewebekontaktflächen (114, 124) gekoppelt ist und dazu ausgelegt ist, dorthin elektrochirurgische Energie zum Behandeln von zwischen den Gewebekontaktflächen (114, 124) gegriffenem Gewebe zuzuführen,
wobei der elektrochirurgische Generator mit dem mindestens einen Temperatursensor gekoppelt ist und dazu ausgelegt ist, erfasste Temperaturdaten davon zu empfangen, wobei der elektrochirurgische Generator eine Steuerung (424) beinhaltet, die, in Echtzeit, ausgelegt ist zum:
Steuern der Zufuhr von elektrochirurgischer Energie zum Gewebe;
Vorhersagen einer Wärmeausbreitung über das erste und das zweite Backenelement hinaus basierend zumindest auf den erfassten Temperaturdaten; und
Modifizieren, wenn bestimmt wird, dass die vorhergesagte thermische Ausbreitung über einer thermischen Schwellenausbreitung liegt, der Zufuhr von elektrochirurgischer Energie zu Gewebe, um die Realisierung der vorhergesagten thermischen Ausbreitung zu hemmen.

2. Elektrochirurgisches System nach Anspruch 1, wobei der mindestens eine Temperatursensor an einem aus dem ersten oder dem zweiten Backenelement angeordnet ist.

3. Elektrochirurgisches System nach Anspruch 2, wobei der mindestens eine Temperatursensor angrenzend an einen Außenumfang des mindestens einen aus dem ersten oder zweiten Backenelement angeordnet ist.

4. Elektrochirurgisches System nach Anspruch 2, wobei der mindestens eine Temperatursensor von einem Außenumfang des mindestens einen aus dem ersten oder zweiten Backenelement nach innen beabstandet ist.

5. Elektrochirurgisches System nach Anspruch 1, wobei der mindestens eine Temperatursensor auf einer von der Endeffektoranordnung unabhängigen Vorrichtung angeordnet ist.

6. Elektrochirurgisches System nach Anspruch 1, wobei die Steuerung dazu ausgelegt ist, die Wärmeausbreitung basierend auf mindestens den erfassten Temperaturdaten und elektrischen Daten, die mit der Energiezufuhr assoziiert sind, vorherzusagen, wobei optional die elektrischen Daten mindestens Impedanzdaten beinhalten.

7. Elektrochirurgisches System nach Anspruch 1, wobei die Steuerung dazu ausgelegt ist, die Wärmeausbreitung basierend auf mindestens den erfassten Temperaturdaten und Fortschrittsstatusinformationen vorherzusagen.

8. Elektrochirurgisches System nach Anspruch 1, wobei die Steuerung dazu ausgelegt ist, die Zufuhr von elektrochirurgischer Energie zu Gewebe zu modifizieren, wobei bestimmt wird, dass die vorhergesagte Wärmeausbreitung über der Schwellenwärmeausbreitung liegt, durch Reduzieren von Leistung, Schalten eines Energieabgabealgorithmus oder Stoppen der Zufuhr von Energie.

9. Elektrochirurgisches System nach Anspruch 1, wobei die Steuerung einen Maschinenlernalgorithmus beinhaltet oder dazu ausgelegt ist, mit diesem zu kommunizieren, der dazu ausgelegt ist, die Vorhersage einer thermischen Ausbreitung zu erleichtern.

10. Elektrochirurgisches System nach Anspruch 1, wobei die Steuerung so ausgelegt ist, dass die Vorhersage der thermischen Ausbreitung für mindestens 3 Sekunden in die Zukunft ist.

## Revendications

1. Système électrochirurgical comprenant :
un ensemble effecteur terminal (100) comprenant des premier (110) et second (120) éléments de mâchoire définissant chacun une surface électriquement conductrice en contact avec le tissu (114, 124),
au moins l'un des premier et second éléments de mâchoire (110, 120) étant mobile par rapport à l'autre entre une position espacée et une position rapprochée pour saisir le tissu entre ses surfaces de contact avec le tissu (114, 124) ;
au moins un capteur de température (150) associé à l'ensemble effecteur terminal (100) ; et
un générateur électrochirurgical (400) couplé électriquement aux surfaces en contact avec les tissus (114, 124) et configuré pour fournir de l'énergie électrochirurgicale à celles-ci afin de traiter les tissus saisis entre les surfaces en contact avec les tissus (114, 124),
le générateur électrochirurgical étant couplé à l'au moins un capteur de température et configuré pour recevoir des données de température détectées à partir de celui-ci, le générateur électrochirurgical comprenant un dispositif de commande (424) configuré, en temps réel, pour :
commander la fourniture d'énergie électrochirurgicale aux tissus ;
prédire la propagation thermique au-delà des premier et second éléments de mâchoire sur la base au moins des données de température détectées ; et
modifier, lorsqu'il est déterminé que la propagation thermique prédite est supérieure à un seuil de propagation thermique, la fourniture d'énergie électrochirurgicale au tissu pour empêcher la réalisation de la propagation thermique prédite.

2. Système électrochirurgical selon la revendication 1, l'au moins un capteur de température étant disposé sur l'un des premier ou second éléments de mâchoire.

3. Système électrochirurgical selon la revendication 2, l'au moins un capteur de température étant disposé adjacent à une périphérie extérieure de l'au moins un des premier ou second éléments de mâchoire.

4. Système électrochirurgical selon la revendication 2, l'au moins un capteur de température étant espacé vers l'intérieur d'une périphérie extérieure de l'au moins un des premier ou second éléments de mâchoire.

5. Système électrochirurgical selon la revendication 1, l'au moins un capteur de température étant disposé sur un dispositif indépendant de l'ensemble effecteur terminal.

6. Système électrochirurgical selon la revendication 1, le dispositif de commande étant configuré pour prédire la propagation thermique sur la base d'au moins les données de température détectées et les données électriques associées à l'alimentation en énergie, éventuellement les données électriques comprenant au moins des données d'impédance.

7. Système électrochirurgical selon la revendication 1, le dispositif de commande étant configuré pour prédire la propagation thermique sur la base d'au moins les données de température détectées et les informations d'état de progression.

8. Système électrochirurgical selon la revendication 1, le dispositif de commande étant configuré pour modifier la fourniture d'énergie électrochirurgicale au tissu, où il est déterminé que la propagation thermique prédite est au-dessus du seuil de propagation thermique, en réduisant la puissance, en commutant un algorithme de fourniture d'énergie, ou en arrêtant la fourniture d'énergie.

9. Système électrochirurgical selon la revendication 1, le dispositif de commande incorporant ou étant configuré pour communiquer avec un algorithme d'apprentissage automatique configuré pour faciliter la prédiction de la propagation thermique.

10. Système électrochirurgical selon la revendication 1, le dispositif de commande étant configuré de telle sorte que la prédiction de la propagation thermique est d'au moins 3 secondes dans le futur.
